(19) Europäisches Patentamt
European Patent Office
Office européen des brevets

(11) Veröffentlichungsnummer: **0 163 888**
**A1**

# (12) EUROPÄISCHE PATENTANMELDUNG

(21) Anmeldenummer: **85104825.6**

(22) Anmeldetag: **20.04.85**

(51) Int. Cl.⁴: **C 07 C 133/12**, C 07 D 311/68, C 07 D 333/66, C 07 D 335/06, C 07 D 209/40, C 07 D 215/42, C 07 D 345/00, C 07 D 409/12, A 61 K 31/155, A 61 K 31/35, A 61 K 31/38

(30) Priorität: **05.05.84 DE 3416695**

(43) Veröffentlichungstag der Anmeldung: **11.12.85 Patentblatt 85/50**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Stegelmeier, Hartmut, Dr., Meide 1d, D-4010 Hilden (DE)**
Erfinder: **Morich, Frank-Joachim, Dr., Untere Bergerheide 15, D-5600 Wuppertal 1 (DE)**
Erfinder: **Knorr, Andreas, Dr., Pahlkestrasse 15, D-5600 Wuppertal 1 (DE)**

**(54) Amidinohydrazone von Tetralin-, Chromon-, Thiochromon- und Tetrahydrochinolin-Derivaten, Verfahren zu ihrer Herstellung sowie ihre Verwendung in Arzneimitteln.**

(57) Die vorliegende Anmeldung betrifft neue Amidinohydrazone der allgemeinen Formel I

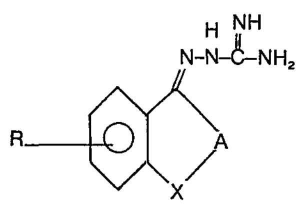

(I)

in welcher R, X und A verschiedene Bedeutungen haben.

Verfahren zu ihrer Herstellung sowie ihre Verwendung in Arzneimitteln, insbesondere in kreislaufbeeinflussenden Arzneimitteln.

0163888

BAYER AKTIENGESELLSCHAFT 5090 Leverkusen, Bayerwerk

Konzernverwaltung RP
Patentabteilung E/Kü-c

# Amidinohydrazone von Tetralin-, Chromon-, Thiochromon- und Tetrahydrochinolin-Derivaten, Verfahren zu ihrer Herstellung sowie ihre Verwendung in Arzneimitteln

Die vorliegende Erfindung betrifft neue Amidinohydrazone von Tetralin-, Chromon-, Thiochromon- und Tetrahydrochinolinderivaten, Verfahren zu ihrer Herstellung sowie ihre Verwendung in Arzneimitteln, insbesondere in Kreislaufbeeinflussenden Arzneimitteln.

Die neuen Amidinohydrazone sind durch folgende allgemeine Formel I gekennzeichnet,

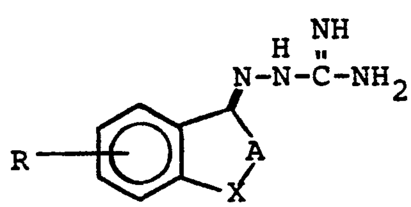

(I)

in welcher

R für Wasserstoff oder ein bis vier gleiche oder verschiedene Substituenten aus der Gruppe

Halogen, Alkyl (1 bis 10 C-Atome) gegebenenfalls substituiert durch $C_1$-$C_6$ Alkylamin, Alkoxy (1 bis 10 C-Atome), Alkylthio (1 bis 10 C-Atome), Alkylsulfinyl (1 bis 10 C-Atome), Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome), -O-$C_1$-$C_6$ Alkylen-$CO_2$-$R_{18}$, wobei $R_{18}$ $C_1$-$C_6$ Alkyl, $C_1$-$C_6$ Alkoxy-$C_1$-$C_6$-alkylen, $C_1$-$C_6$ Alkoxycarbonyl, N-$C_1$-$C_6$ Alkylcarbamat bedeutet, oder für $NR_{19}R_{20}$ steht, wobei $R_{19}$ und $R_{20}$ gleich oder verschieden H, CO-$C_1$-$C_6$-alkylamin, CONH-Heteroaryl, CO-$C_1$-$C_6$ Alkyl darstellen,

X für $CR^1R^2$, O, $S(O)_n$ (n=0,1,2), $NR^3$, Se steht und

A für eine Bindung, $CR^4R^5$, $CR^6R^7$-$CR^8R^9$, $CR^{10}R^{11}$-$CR^{12}R^{13}$-$CR^{14}R^{15}$, $CR^{16}$=$CR^{17}$ CO, C=NOH steht, worin

$R^1$, $R^2$, $R^4$-$R^{17}$ gleich oder verschieden für Wasserstoff oder Substituenten aus der Gruppe Halogen, Alkyl (1 bis 10 C-Atome), Alkoxy (1 bis 10 C-Atome), Alkylthio (1 bis 10 C-Atome), Alkylsulfinyl (1 bis 10 C-Atome), Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio

(1 bis 5 C-Atome), Amino, Monoalkylamino (1 bis 5 C-Atome), Dialkylamino (jeweils 1 bis 5 C-Atome) steht oder für einen Aromaten oder Heteroaromaten steht, der gegebenenfalls mit einem bis 5 gleiche oder verschiedene Substituenten aus der Gruppe Wasserstoff, Halogen, Alkyl (1 bis 10 C-Atome), Alkoxy (1 bis 10 C-Atome), Alkylthio (1 bis 10 C-Atome), Alkylsulfinyl (1 bis 10 C-Atome), Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome) substituiert ist

$R^3$ für Wasserstoff, Alkyl (1 bis 10 C-Atome) Aryl, Acyl (1 bis 10 C-Atome) oder Alkansulfonyl (1 bis 10 C-Atome), gegebenenfalls substituiert mit einem bis 5 gleiche oder verschiedene Substituenten aus der Gruppe Wasserstoff, Halogen, Alkyl (1 bis 10 C-Atome), Alkoxy (1 bis 10 C-Atome), Alkylthio (1 bis 10 C-Atome), Alkylsulfinyl (1 bis 10 C-Atome), Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome) steht,

in Form von Isomeren, Isomerengemischen, Racematen und optischen Antipoden sowie ihre pharmazeutisch unbedenklichen Salze.

Als Salze seien beispielsweise genannt Hydrochloride, Hydrogensulfate, Sulfate, Hydrogenphosphate, Acetate, Maleate, Benzoate, Citronate, Tartrate oder Lactate.

Bevorzugt sind Verbindungen der allgemeinen Formel I, in der

A eine Einfachbindung, $C_1$-$C_8$ Alkylen, $C_2$-$C_6$ Alkenylen, CO, C=NOH, $C_2$-$C_6$ Alkenylen, substituiert durch Phenyl, Chlorphenyl, Nitrophenyl, Aminophenyl, Mono-$C_1$-$C_3$-alkylamin, Di-$C_1$-$C_3$-alkylamin, Aryl-$C_1$-$C_4$-alkylen, Phenoxy-$C_1$-$C_4$-alkylen darstellt, wobei der Phenoxyrest gegebenenfalls substituiert ist durch Halogen, Nitro, $C_1$-$C_4$-Alkyl, $C_1$-$C_4$ Alkoxy, Cyano, Hydroxy, Amino, Mono-$C_1$-$C_3$-alkylamin, Di-$C_1$-$C_3$-alkylamin, oder Di-$C_1$-$C_3$-alkylamino-$C_2$-$C_6$-alkylen,

R H, Halogen, OH, Nitro, Amino, $C_1$-$C_3$-Monoalkylamino, $C_1$-$C_3$ Dialkylamino, $C_1$-$C_6$ Alkyl gegebenenfalls substituiert durch Morpholin, Piperidin, Pyrrolidin; $C_1$-$C_6$ Alkoxy, Cyano, $-O-CH_2-CO_2R_{18}$, wobei $R_{18}$ $C_1$-$C_4$ Alkyl, $C_1$-$C_4$ Alkylcarbamat darstellt, $NR_{19}R_{20}$ wobei $R_{19}$ und $R_{20}$ gleich oder verschieden H, CO-Morpholin, CO-Piperidin, CO-Pyrrolidin, CONH-Pyridyl oder CO-$C_1$-$C_4$ Alkyl bedeutet, und

X S, Se, O, $SO_2$, $CR_1R_2$ oder $NR_3$ darstellt, wobei $R_1$ und $R_2$ gleich oder verschieden Wasserstoff, $C_1$-$C_6$ Alkyl oder Phenyl bedeuten, wobei der Phenylrest gegebenenfalls durch Halogen, $C_1$-$C_6$-Alkyl, $C_1$-$C_6$ Alkoxy, Hydroxy, Nitro, Cyano, Amino, Mono-$C_1$-$C_4$-alkylamino oder Di-$C_1$-$C_4$alkylamino substituiert ist und wobei der Rest $R_3$ Wasserstoff, Acyl, Phenyl, Naphthyl oder Pyridyl darstellt und die Phenyl-, Naphthyl- oder Pyridylreste gegebenenfalls substituiert sind durch Halogen, $C_1$-$C_6$ Alkyl, $C_1$-$C_6$-Alkoxy, Hydroxy, Cyano, Amino, Mono-$C_1$-$C_4$-alkylamino und/oder Di-$C_1$-$C_4$-alkylamino.

Insbesondere kommen Verbindungen der allgemeinen Formel I in Betracht, in denen

A $C_1$-$C_5$ Alkylen, Propenylen, CO, C=NOH, CH=C-Phenyl, CH=C-Chlorphenyl, Phenyl-$C_1$-$C_3$-alkylen, Dichlorphenoxy-$C_1$-$C_3$-alkylen oder $(CH_3)_2N$-$C_3$-alkylen darstellt,

X S, Se, O, $SO_2$, $CR_1R_2$ oder $NR_3$ bedeutet, wobei $R_3$ Wasserstoff, Phenyl, Nitrophenyl oder Ethoxycarbonyl darstellt und wobei $R_1$ und $R_2$ gleich oder verschieden Wasserstoff oder Phenyl bedeuten,

R Wasserstoff, Chlor, Hydroxy, Nitro, Amino, $C_1$-$C_3$-Alkyl gegebenenfalls substituiert durch Morpholino, $C_1$-$C_3$ Alkoxy, $-O-CH_2CO_2R_{18}$, wobei $R_{18}$ $C_1$-$C_4$ Alkyl, $C_1$-$C_3$ Alkoxy-$C_1$-$C_4$-alkylen, $C_1$-$C_3$-Alkoxycarbonyl oder N-$C_1$-$C_3$ Alkylcarbamat darstellt; oder N $R_{19}R_{20}$

bedeutet, wobei $R_{19}$ und $R_{20}$ gleich oder verschieden Wasserstoff, CO-Morpholino, CO-NH-Pyridyl oder Acyl darstellt.

Die erfindungsgemäßen Verbindungen der allgemeinen Formel I können nach bekannten Verfahren hergestellt werden. Man erhält sie z.B. wenn man Ketone der allgemeinen Formel (II)

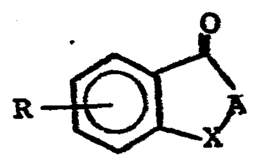

(II)

in welcher R, X und A die oben angegebene Bedeutung besitzen in Gegenwart von Verdünnungsmitteln, vorzugsweise in Gegenwart katalytischer Mengen Säure bei Temperaturen zwischen 0 und 150°C, vorzugsweise zwischen 40 und 120°C mit Aminoguanidin der Formel

$$H_2\text{-NH-}\overset{\overset{\large NH}{\|}}{C}\text{-}NH_2 \qquad \text{(III)}$$

vorzugsweise in Form eines Säureadditionssalzes umsetzt.

Die Reaktionsprodukte fallen im allgemeinen aus, werden abgesaugt und mit niederen Alkoholen vorzugsweise mit Ethanol gewaschen.

Bei der Umsetzung von Verbindungen der Formel (II) mit Aminoguanidin der Formel (III) vorzugsweise in Form ihrer Salze (z.B. Hydrochloride oder Carbonate) setzt man vorzugsweise inerte, polare, organische Lösungsmittel ein, in welchen sich die Komponenten II und III gut lösen und das Reaktionsprodukt in schwer löslicher Form ausfällt. Als für die Umsetzung brauchbare Lösungsmittel seien beispielhaft genannt:

Niedere Alkanole wie Methanol, Ethanol und i-Propanol, insbesondere Ethanol. Ketone wie Aceton, Methylethylketon. Säurenitrile wie z.B. Acetonitril und Propionitril. Ether wie Dioxan, Tetrahydrofuran.

Die als Ausgangsprodukte benötigten Ketone sind in der Regel bekannt oder können nach bekannten Verfahren hergestellt werden (Houben-Weyl, Methoden der organischen Chemie).

Die erfindungsgemäßen Verbindungen zeigen ein nicht vorhersehbares und wertvolles pharmakologisches Wirkungsspektrum. Sie interagieren in spezifischer Weise mit der Bindungsstelle der Digitalisglykoside bzw. ihrer endogenen Analoga ("Natriuretisches Hormon", "Endogenes Digitalis"). Dabei zeigt ein Teil der Verbindungen die gleichen Wirkungen (Agonisten), während andere die Wirkung von Digitalis aufheben (Antagonisten).

Aufgrund dieser Eigenschaften können diese Substanzen sowohl den Blutdruck senken bzw. bei Digitalisvergiftungen als Antidot eingesetzt werden (Antagonisten), als auch den Blutdruck erhöhen, die Kontraktionskraft des Herzens steigern und die renale Kochsalzausscheidung fördern (Agonisten).

Die erfindungsgemäßen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol,

Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermitteln (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbessern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation, größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Beispiel 1

### 6-Methoxy-1-tetralon-amidinohydrazon-hydrochlorid

21,1 g (0,12 Mol) 6-Methoxytetralon wurden in 220 ml Methanol gelöst und zu einer Lösung aus 18 g (0,132 Mol) Aminoguanidinhydrogencarbonat in Methanol/HCl (pH 2) gegeben. Man läßt über Nacht bei Raumtemperatur rühren, saugt das Rohprodukt ab und kristallisiert mit Aceton. Ausbeute: 25,2 g (65,4 %) Fp.: = 245-47°C (Zers.).

## Beispiel 2

### 6-Methoxy-2-methyl-4-thiochromanonamidinohydrazon-hydrochlorid

4,1 g (0,02 Mol) 6-Methoxy-2-methyl-4-thiochromanon wurden unter Erwärmen in 50 ml Methanol gelöst und mit einer Lösung aus 3 g (0,022 Mol) Aminoguanidinhydrogencarbonat in Methanol/HCl (pH 2) versetzt. Man läßt über Nacht bei Raumtemperatur stehen und kocht anschließend 30 Minuten unter Rückfluß. Dann wird die Hälfte des Lösemittels am Rotationsverdampfer abgezogen, die entstandenen Kristalle abgesaugt und mit Ether gewaschen. Ausbeute: 5,8 g (68,1 %) Fp.: 213-15°C.

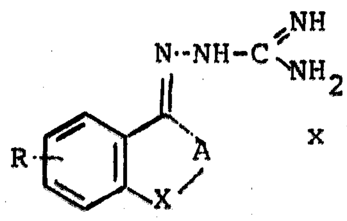 x HCl

| Beispiel | R | A | X | Fp(°C) | analog Beispiel |
|---|---|---|---|---|---|
| 3 | H | C=O | S | 247 | 1 |
| 4 | $6-OCH_2CO_2CH(CH_3)_2$ | $CH=C-CH_3$ | O | 180-185 | 2 |
| 5 | 5-Cl | $CH_2-CH_2$ | O | 290 | 2 |
| 6 | $6-OCH_2CO_2CH_2CH_2-OCH_3$ | $CH=C-CH_3$ | O | 225 | 2 |
| 7 | $5-NO_2$, $6-OCH_2CO_2-C_2H_5$ | $CH=C-CH_3$ | O | 155 | 2 |
| 8 | $5-OCH_3$ | $CH=C-C_6H_5$ | S | > 250 | 2 |
| 9 | $5-CH_2-N$ 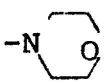 | $CH=C-CH_3$ | O | 132 | 2 |
| | $6-OCH_2CO_2C_2H_5$ | | | | |
| 10 | H | C=O | $N-C_6H_5$ | 240(Zers.) | 2 |
| 11 | $7-OCH_3$ | CH=C- 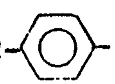 -Cl | S | 238-240 | 2 |
| 12 | $5-OCH_3$ | $CH=C-CH_3$ | S | 193-195 | 2 |
| 13 | $5-CO_2CH_3$ | CH=C- 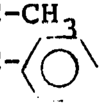 | S | 232 | 2 |
| 14 | H | C=O | N- 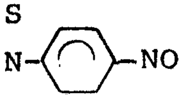 $-NO_2$ | 287(Zers.) | 2 |
| 15 | H | $CH_2CH_2$ | $CH_2$ | 160-61 | 1 |

| Beispiel | R | A | X | Fp(°C) | analog Beispiel |
|---|---|---|---|---|---|
| 16 | 6-$NH_2$ | $CH=C-CH_3$ | S | >260 | 2 |
| 17 | 8-NH-CO-N 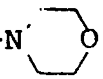 | $CH=C-CH_3$ | S | 215 | 2 |
| 18 | 7-Cl | $CH=C-CH_3$ | O | - | 2 |
| 19 | 7-$OCH_3$ | CH=C- 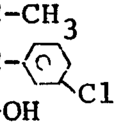 | O | - | 2 |
| 20 | H | C=N-OH | S | - | 2 |
| 21 | 6-NHCO-NH- 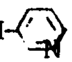 | $CH=C-CH_3$ | S | 222 | 2 |
| 22 | 6-$OCH_2CO_2C_2H_5$ | $CH=C-CH_3$ | O | 213-215 | 2 |
| 23 | 5-$OCH_2CO_2C_2H_5$ | $CH=C-CH_3$ | O | 172 | 2 |
| 24 | H | $CH=C-CH_3$ | Se | 140 | 2 |
| 25 | 5-$NHCOCH_3$ | $CH_2-CH_2$ | S | 273-75 | 1 |
| 26 | 7-Cl | $CH_2-CH_2$ | $SO_2$ | 248-50 | 1 |
| 27 | 5-$CH_3$, 8-$NH_2$ | $CH_2-CH_2$ | S | 263-65 | 2 |
| 28 | 6-$OCH_2CO_2CH_3$ | $CH_2-CH_2$ | S | 220-22 | 1 |
| 29 | 5-OH | $CH_2-CHCH_3$ | S | 158-60 | 1 |
| 30 | 5-$OCH_3$, 8-$CH_3$ | $CH_2-CHCH_3$ | S | 145-147 | 2 |
| 31 | 5-$NO_2$, 7-Cl | $CH_2-CH_2$ | S | 270 | 1 |
| 32 | 7-OH | $CH_2$-CH- 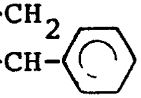 | O | 263-65 | 2 |

| Beispiel | R | A | X | Fp(°C) | analog Beispiel |
|---|---|---|---|---|---|
| 33 | 7-Cl | $CH_2-CH_2$ | O | 273-75 | 1 |
| 34 | 6-Cl | $CH_2-CH_2$ | NH | 255-58 | 1 |
| 34 | 6-Cl | $CH_2-CH_2$ | NH | 255-58 | 1 |
| 35 | $5-OCH_3$ | $CH_2$ | $CH_2$ | 270-72 | 1 |
| 36 | H | $CH_2CH-CH(CH_3)_2$ | O | 243-45 | 1 |
| 37 | H | $(CH_3)_2N-CH_2-$ $CH-CH_2$ | O | 228-30 | 2 |
| 38 | 8-OH | $CH_2-CH-CH(CH_3)_2$ | O | 278-80 | 1 |
| 39 | H | $CH_2-CH_2$ | NH | 245-47 | 1 |
| 40 | $7-OCONHCH_3$ | $CH_2-CH-$ 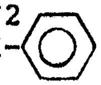 | O | 220 | 1 |
| 41 | H | $CH_2$ | C 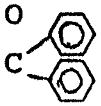 | 265 | 1 |
| 42 | H | $CH_2$ | CH- 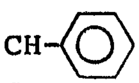 | 250 | 1 |
| 43 | 6-Cl | $CH_2-CH_2$ | S | 228-30 | 1 |
| 44 | 6-OH | $CH_2CH-CH_3$ | O | 233-35 | 2 |
| 45 | $7-CH_3$ | $CH_2-C<\begin{matrix}CH_3\\CH_3\end{matrix}$ | O | 205-07 | 1 |
| 46 | H | $CH_2-CH_2$ | O | 268-70 | 1 |

| Beispiel | R | A | X | Fp(°C) | analog Beispiel |
|---|---|---|---|---|---|
| 47 | 6-Cl | $CH_2$ | $CH_2$ | >280 | 1 |
| 48 | H | $CH_2-C(CH_3)_2$ | O | 208-10 | 2 |
| 49 | 6-Cl | $CH_2-CH_2$ | $N-COC_2H_5$ | 280-81 | 1 |
| 50 | H | $CH(CH_3)-CH_2$ | $CH_2$ | 172-74 | 2 |
| 51 | H | $CH_2-CHO-$ 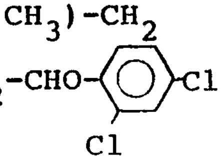 | $CH_2$ | 198-202 | 2 |
| 52 | $4-CH_3$, $7-CH_3$ | $CH_2$ | $CH_2$ | 235-36 | 1 |
| 53 | H | $CH_2$ | $CH_2$ | 270-72 | 1 |
| 54 | $5-OCH_3$, $6-OCH_3$ | $CH_2$ | $CH_2$ | 210-11 | 1 |
| 55 | $5-CH_3$, $7-CH_3$ | $CH_2-CH_2$ | $CH_2$ | 233-35 | 1 |
| 56 | $5-OCH_3$ | $CH_2-CH_2$ | $CH_2$ | 215-16 | 1 |

Patentansprüche:

1. Amidinohydrazone der allgemeinen Formel I

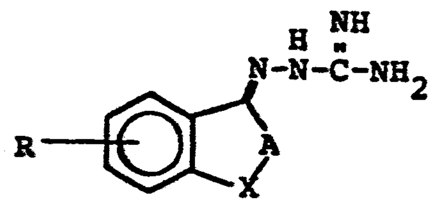

(I)

in welcher

R für Wasserstoff oder ein bis vier gleiche oder verschiedene Substituenten aus der Gruppe Halogen, $C_1$-$C_{10}$ Alkyl, gegebenenfalls substituiert durch $C_1$-$C_6$ Alkylamin, $C_1$-$C_{10}$ Alkoxy $C_1$-$C_{10}$ Alkylthio, $C_1$-$C_{10}$ Alkylsulfinyl, Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome), -O-$C_1$-$C_6$ Alkylen-$CO_2$-$R_{18}$, wobei $R_{18}$ $C_1$-$C_6$ Alkyl, $C_1$-$C_6$ Alkoxy-$C_1$-$C_6$-alkylen, $C_1$-$C_6$ Alkoxycarbony oder N-$C_1$-$C_6$ Alkylcarbamat bedeutet, oder für $NR_{19}R_{20}$ steht, wobei $R_{19}$ und $R_{20}$ gleich oder verschieden H, CO-$C_1$-$C_6$-alkylamin, CONH-Heteroaryl, CO-$C_1$-$C_6$ Alkyl darstellen,

X für $CR^1R^2$, O, $S(O)_n$ (n=0,1,2), $NR^3$, Se steht und

A für eine Bindung, $CR^4R^5$, $CR^6R^7$-$CR^8R^9$, $CR^{10}R^{11}$-$CR^{12}R^{13}$-$CR^{14}R^{15}$, $CR^{16}$=$CR^{17}$ CO, C=NOH steht, worin

$R^1$, $R^2$, $R^4$-$R^{17}$ gleich oder verschieden für Wasserstoff oder Substituenten aus der Gruppe Halogen, $C_1$-$C_{10}$ Alkyl, $C_1$-$C_{10}$ Alkoxy, $C_1$-$C_{10}$-Alkylthio, $C_1$-$C_{10}$Alkyl -sulfinyl, Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome) steht oder für einen Aromaten oder Heteroaromaten steht, der gegebenenfalls mit einem bis 5 gleiche oder verschiedene Substituenten aus der Gruppe Wasserstoff, Halogen, $C_1$-$C_{10}$ Alkyl, $C_1$-$C_{10}$ Alkoxy, $C_1$-$C_{10}$ Alkylthio, $C_1$-$C_{10}$ Alkylsulfinyl, Cyano, Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome) substituiert ist,

$R^3$ für Wasserstoff, $C_1$-$C_{10}$Alkyl, Aryl, $C_1$-$C_{10}$ Acyl, oder Alkansulfonyl (1 bis 10 C-Atome), gegebenenfalls substituiert mit einem bis 5 gleiche oder verschiedene Substituenten aus der Gruppe Wasserstoff, Halogen, $C_1$-$C_{10}$ Alkyl, $C_1$-$C_{10}$ Alkoxy, $C_1$-$C_{10}$ Alkylthio, $C_1$-$C_{10}$ Alkylsulfinyl, Cyano,

Hydroxy, Nitro, Mono- oder Polyfluoralkyl (1 bis 5 C-Atome), Mono- oder Polyfluoralkoxy (1 bis 5 C-Atome), Mono- oder Polyfluoralkylthio (1 bis 5 C-Atome), Amino, Monoalkylamino (1 bis 5 C-Atome), Dialkylamino (jeweils 1 bis 5 C-Atome) steht,

in Form von Isomeren, Isomerengemischen, Racematen und optischen Antipoden sowie ihre pharmazeutisch unbedenklichen Salze.

2. Verbindungen gemäß Formel I des Anspruchs 1, in welchen

A eine Einfachbindung, $C_1$-$C_8$ Alkylen, $C_2$-$C_6$ Alkenylen, CO, C=NOH, $C_2$-$C_6$ Alkenylen, substituiert durch Phenyl, Chlorphenyl, Nitrophenyl, Aminophenyl, Mono-$C_1$-$C_3$-alkylamin, Di-$C_1$-$C_3$-alkylamin, Aryl-$C_1$-$C_4$-alkylen, Phenoxy-$C_1$-$C_4$-alkylen darstellt, wobei der Phenoxyrest gegebenenfalls substituiert ist durch Halogen, Nitro, $C_1$-$C_4$-Alkyl, $C_1$-$C_4$ Alkoxy, Cyano, Hydroxy, Amino, Mono-$C_1$-$C_3$-alkylamin, Di-$C_1$-$C_3$-alkylamin, oder Di-$C_1$-$C_3$-alkylamino-$C_2$-$C_6$-alkylen,

R H, Halogen, OH, Nitro, Amino, $C_1$-$C_3$-Monoalkylamino, $C_1$-$C_3$ Dialkylamino, $C_1$-$C_6$ Alkyl gegebenenfalls substituiert durch Morpholin, Piperidin, Pyrrolidin; $C_1$-$C_6$ Alkoxy, Cyano, $-O-CH_2-CO_2R_{18}$, wobei $R_{18}$ $C_1$-$C_4$ Alkyl, $C_1$-$C_4$ Alkylcarbamat darstellt, $NR_{19}R_{20}$ wobei $R_{19}$ und $R_{20}$ gleich oder verschieden H, CO-Morpholin, CO-Piperidin, CO-Pyrrolidin, CONH-Pyridyl oder CO-$C_1$-$C_4$ Alkyl bedeutet, und

X S, Se, O, $SO_2$, $CR_1R_2$ oder $NR_3$ darstellt, wobei $R_1$ und $R_2$ gleich oder verschieden Wasserstoff, $C_1$-$C_6$ Alkyl oder Phenyl bedeuten, wobei der Phenylrest gegebenenfalls durch Halogen, $C_1$-$C_6$-Alkyl, $C_1$-$C_6$ Alkoxy, Hydroxy, Nitro, Cyano, Amino, Mono-$C_1$-$C_4$-alkylamino oder Di-$C_1$-$C_4$-alkylamino substituiert ist und wobei der Rest $R_3$ Wasserstoff Acyl, Phenyl, Naphthyl oder Pyridyl darstellt und die Phenyl-, Naphthyl- oder Pyridylreste gegebenenfalls substituiert sind durch Halogen, $C_1$-$C_6$ Alkyl, $C_1$-$C_6$-Alkoxy, Hydroxy, Cyano, Amino, Mono-$C_1$-$C_4$-alkylamino und/oder Di-$C_1$-$C_4$-alkylamino.

3. Verbindungen gemäß Formel I des Anspruchs 1, in welchen

A $C_1$-$C_5$ Alkylen, Propenylen, CO, C=NOH, CH=C-Phenyl, CH=C-Chlorphenyl, Phenyl-$C_1$-$C_3$-alkylen, Dichlorphenoxy-$C_1$-$C_3$-alkylen oder $(CH_3)_2N$-$C_3$-alkylen darstellt,

X S, Se, O, $SO_2$, $CR_1R_2$ oder $NR_3$ bedeutet, wobei $R_3$ Wasserstoff, Phenyl, Nitrophenyl oder Ethoxycarbonyl darstellt und wobei $R_1$ und $R_2$ gleich oder verschieden Wasserstoff oder Phenyl bedeuten,

R Wasserstoff, Chlor, Hydroxy, Nitro, Amino, $C_1$-$C_3$-Alkyl gegebenenfalls substituiert durch Morpholino, $C_1$-$C_3$ Alkoxy, $-O-CH_2CO_2R_{18}$, wobei $R_{18}$

$C_1$-$C_4$ Alkyl, $C_1$-$C_3$ Alkoxy-$C_1$-$C_4$-alkylen, $C_1$-$C_3$-Alkoxycarbonyl oder N-$C_1$-$C_3$ Alkylcarbamat darstellt; oder N $R_{19}R_{20}$ bedeutet, wobei $R_{19}$ und $R_{20}$ gleich oder verschieden Wasserstoff, CO-Morpholino, CO-NH-Pyridyl oder Acyl darstellt.

4. Verfahren zur Herstellung der Verbindungen gemäß Anspruchen 1 bis 3, dadurch gekennzeichnet, daß man Ketone der allgemeinen Formel (II)

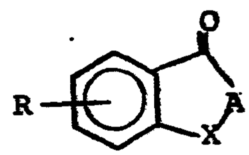

(II)

in welcher R, X und A die in den Ansprüchen 1 bis 3 angegebene Bedeutung besitzen in Gegenwart von Verdünnungsmitteln, bei Temperaturen zwischen 0 und 150°C, mit Aminoguanidin der Formel (III)

$$H_2\text{-NH-}\overset{\overset{\text{NH}}{\|}}{\text{C}}\text{-NH}_2 \quad \text{(III)}$$

umsetzt.

5. Verfahren gemäß Anspruch 4, dadurch gekennzeichnet, daß man die Reaktion in Gegenwart katalytischer Mengen an Säure durchführt.

6. Verfahren gemäß Anspruch 4, dadurch gekennzeichnet, daß man die Umsetzung zwischen 40 und 120°C durchführt.

7. Arzneimittel enthaltend als Wirkstoff eine Verbindung der Ansprüche 1 bis 3.

8. Amidinohydrazone der Ansprüche 1 bis 3 zur Bekämpfung von Erkrankungen.

9. Verwendung der Verbindungen gemäß Ansprüchen 1 bis 3 zur Bekämpfung von Erkrankungen.

10. Verwendung der Verbindungen gemäß Ansprüchen 1 bis 3 zur Bekämpfung des Bluthochdruckes, der Digitalisvergiftung, des Blutniederdruckes, zur Steigerung der Kontraktionskraft des Herzens oder zur Förderung der Kochsalzausscheidung.

Europäisches Patentamt

**EUROPÄISCHER RECHERCHENBERICHT**

0163888

Nummer der Anmeldung

EP 85 10 4825

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-1 083 730 (FARBENFABRIKEN BAYER AG) * Ansprüche 1-15; Beispiele; Seite 2, Zeilen 37-45 * | 1-10 | C 07 C 133/12<br>C 07 D 311/68<br>C 07 D 333/66<br>C 07 D 335/06<br>C 07 D 209/40<br>C 07 D 215/42<br>C 07 D 345/00<br>C 07 D 409/12<br>A 61 K 31/155<br>A 61 K 31/35<br>A 61 K 31/38<br>A 61 K 31/40<br>A 61 K 31/47<br>A 61 K 31/33 |
| X | DE-A-2 736 064 (BAYER AG) * Anspruch 1; Beispiele 1-3, 5, 7, 10, 15 * | 1-9 | |
| X | US-A-4 076 726 (H.J. PANNEMAN et al.) * Ansprüche; Beispiele II, VIII, XVI-XX, XXIII-XXVI, XXVIII; Spalte 1, Zeile 65 - Spalte 2, Zeile 30; Spalte 4, Zeilen 10-17; Spalte 5, Zeilen 3-17 * | 1-4,7, 10 | **RECHERCHIERTE SACHGEBIETE (Int. Cl.4)** |
| X | DE-A-2 020 230 (AGENCE NATIONALE DE VALORISATION DE LA RECHERCHE) * Ansprüche; Beispiele 3, 4 * | 1-4,6-9 | C 07 C 123/00<br>C 07 C 133/00<br>C 07 D 209/00<br>C 07 D 215/00<br>C 07 D 311/00<br>C 07 D 333/00<br>C 07 D 335/00<br>C 07 D 345/00<br>C 07 D 409/12 |

Der vorliegende Recherchenbericht wurde für alle Patentansprüche erstellt.

| Recherchenort | Abschlußdatum der Recherche | Prüfer |
|---|---|---|
| BERLIN | 30-07-1985 | HASS C V F |

**KATEGORIE DER GENANNTEN DOKUMENTEN**

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503. 03 82